# EUROPÄISCHE PATENTANMELDUNG

(11) **EP 4 748 336 A1**
(43) Veröffentlichungstag der Anmeldung: **27.05.2026**
(21) Anmeldenummer: 25216157.5
(22) Anmeldetag: 17.11.2025
(51) Int. Cl.: A61B 34/30, A61B 90/20

(54) **MEDIZINISCHES MIKROSKOPIESYSTEM ZUR AUFNAHME EINES ANATOMISCHEN ZIELGEBIETS EINES PATIENTEN**

(30) Priorität: 22.11.2024 DE 102024134506
(71) Anmelder: B. Braun New Ventures GmbH, 79110 Freiburg im Breisgau (DE)
(72) Erfinder: SARVESTANI, Amir, 79104 Freiburg (DE)
(74) Vertreter: Winter, Brandl - Partnerschaft mbB

(57) **Zusammenfassung**

Die vorliegende Offenbarung betrifft ein Mikroskopiesystem (10) zur Aufnahme eines anatomischen Zielgebiets (11, 62) eines Patienten (12), wobei oberhalb des anatomischen Zielgebiets (11, 62) eine zylindrische Hülse (13, 20, 30, 50, 61) angeordnet ist, mit einer Aufnahmeeinheit (14, 63, 64), einer Gelenkarmrobotereinheit (15), einer Auswerteeinheit (16), die eingerichtet ist, in dem aufgenommenen Bild eine Abweichung der Kontur (21, 31, 40, 51) der Stirnseite von einer Sollkontur einer Stirnseite der zylindrischen Hülse (13, 20, 30, 50, 61) zu bestimmen; wobei die Auswerteeinheit (16) eingerichtet ist, auf Basis der bestimmten Abweichung einen Verfahrbefehl zur Korrektur einer Pose der Gelenkarmrobotereinheit (15) zu bestimmen, und wobei die Gelenkarmrobotereinheit (15) eingerichtet ist, zumindest eine Achse (41, 42) der Gelenkarmrobotereinheit (15) auf Basis des bestimmten Verfahrbefehls zu bewegen. Zudem betrifft die Offenbarung eine Verwendung eines einer Gelenkarmeinheit und/oder einer Aufnahmeeinheit (14, 63, 64) in einem solchen Mikroskopiesystem (10).

## Beschreibung

### Technisches Gebiet

Die vorliegende Offenbarung betrifft ein medizinisches Mikroskopiesystem zur Aufnahme eines anatomischen Zielgebiets eines Patienten in einem medizinischen Operationsbereich sowie eine Verwendung einer Gelenkarmrobotereinheit und/oder einer Aufnahmeeinheit in einem solchen medizinischen Mikroskopiesystem.

### Technischer Hintergrund

Mikroskopiesysteme sind aus dem Stand der Technik grundsätzlich bekannt und werden in medizinischen Operationsbereichen u.a. zur Aufnahme eines anatomischen Zielgebiets bzw. zur Aufnahme eines Patienten bzw. eines Teils des Patienten, beispielsweise von freigelegten Organen eingesetzt.

Dabei kann für eine Operation ein Zugangsport verwendet werden. Der Zugangsport wird dabei im Regelfall am Patienten über einer geöffneten Stelle des Körpers bzw. auf einer Körperoberfläche angeordnet. Der Zugangsport wird im Regelfall über eine zylindrische Hülse realisiert. Ein Chirurg operiert dann durch diesen Zugangsport beispielsweise ein freigelegtes Organ. Hierbei blickt er durch den Zugangsport. Er kann dabei durch ein Mikroskop unterstützt werden, das durch den Zugangsport den zu operierenden Bereich (z.B. Organ) und dessen Umgebung (z.B. Gewebe oder Arterien) aufnimmt und zeitgleich an einem Bildschirm das aufgenommene Bild darstellt.

In diesem Zusammenhang hat sich nun herausgestellt, dass ein weiterer Bedarf besteht, ein medizinisches Mikroskopiesystem zur Aufnahme eines anatomischen Zielgebiets eines Patienten in einem medizinischen Operationsbereich bereitzustellen.

### Zusammenfassung der vorliegenden Offenbarung

Es ist daher Aufgabe der vorliegenden Offenbarung ein medizinisches Mikroskopiesystem zur Aufnahme eines anatomischen Zielgebiets eines Patienten in einem medizinischen Operationsbereich bereitzustellen, insbesondere ist es Aufgabe der vorliegenden Offenbarung, ein effizientes medizinisches Mikroskopiesystem zur Aufnahme eines anatomischen Zielgebiets in einem medizinischen Operationsbereich bereitzustellen, das eine genaue Ausrichtung des medizinischen Mikroskopiesystems in Bezug auf das Objekt ermöglicht.

Die Aufgabe der vorliegenden Offenbarung wird durch ein medizinisches Mikroskopiesystem gemäß Anspruch 1 gelöst. Vorteilhafte Ausgestaltungen sind Gegenstand der Unteransprüche.

Ein erster Aspekt der vorliegenden Offenbarung betrifft ein medizinisches Mikroskopiesystem zur Aufnahme eines anatomischen Zielgebiets eines Patienten in einem medizinischen Operationsbereich, wobei oberhalb des anatomischen Zielgebiets bzw. auf einer Oberfläche des anatomischen Zielgebiets eine zylindrische Hülse angeordnet ist, mit einer Aufnahmeeinheit, die eingerichtet ist, ein Bild des anatomischen Zielgebiets durch die zylindrische Hülse aufzunehmen, wobei das Bild zumindest einen Teilabschnitt einer Kontur einer Stirnseite der zylindrischen Hülse umfasst, einer Gelenkarmrobotereinheit, wobei die Aufnahmeeinheit an der Gelenkarmrobotereinheit angeordnet ist, und einer Auswerteeinheit, die eingerichtet ist, in dem aufgenommenen Bild eine Abweichung des zumindest einen Teilabschnitts der Kontur der Stirnseite von einer Sollkontur einer Stirnseite der zylindrischen Hülse zu bestimmen, wobei die Auswerteeinheit eingerichtet ist, auf Basis der bestimmten Abweichung einen Verfahrbefehl zur Korrektur einer Pose bzw. Position und/oder Orientierung der Gelenkarmrobotereinheit zu bestimmen, sodass die Abweichung reduziert wird, und wobei die Gelenkarmrobotereinheit eingerichtet ist, zumindest eine Achse der Gelenkarmrobotereinheit auf Basis des bestimmten Verfahrbefehls zu bewegen, um eine Ausrichtung der Aufnahmeeinheit zu ermöglichen, sodass die Abweichung reduziert wird.

Der Begriff anatomisches Zielgebiet ist vorliegend breit zu verstehen und meint einen Teil eines Patienten. Das anatomische Zielgebiet kann beispielsweise ein Organ, ein Knochen oder ein Gewebe eines Patienten darstellen. Das Objekt liegt vorzugsweise unter einer Hautoberfläche eines Patienten, wobei die Hautoberfläche bereits geöffnet wurde.

Der Begriff Patient meint vorliegend vorzugsweise einen Menschen oder ein Tier.

Der Begriff Aufnahmeeinheit meint vorliegend eine bildgebende Vorrichtung, die in der Lage ist, ein zweidimensionales oder dreidimensionales Bild aufzunehmen und zur weiteren Verarbeitung, beispielsweise zur Darstellung an einem Bildschirm, zur Verfügung zu stellen. Die Aufnahmeeinheit kann dabei beispielsweise ein Stereomikroskop umfassen. Die Aufnahmeeinheit kann vorzugsweise eingerichtet sein, das Bild zeitaktuell aufzunehmen. Das Bild kann beispielsweise in Echtzeit an dem Bildschirm dargestellt werden. Echtzeit meint hier eine Verzögerung im Millisekundenbereich.

Der Begriff medizinischer Operationsbereich meint vorliegend insbesondere einen Operationssaal.

Der Begriff zylindrische Hülse für einen Zugangsport meint vorliegend insbesondere eine Hohlzylinder, der bei einer Operation auf eine offene Stelle am Körper angeordnet wird, durch die ein Chirurg während einer Operation Instrumente (z.B. Skalpell) einführen kann. Derartige Operation werden auch als minimalinvasive Operationen bezeichnet. Eine derartige Hülse kann auch als Trokarhülse bezeichnet werden. Vorzugsweise kann das medizinische Mikroskopiesystem eine zylindrische Hülse für einen Zugangsport umfassen. Vorzugsweise kann die zylindrische Hülse eingerichtet sein, über einem anatomischen Zielgebiet angeordnet zu werden.

Der Begriff Gelenkarmrobotereinheit meint vorliegend insbesondere einen Gelenkarmroboter mit mehreren beweglichen Achsen, einer Steuerung sowie Drehgebern zur Erfassung einer Position und/oder Orientierung des Endeffektors. Der Endeffektor ist vorliegend die Aufnahmeeinheit. Das heißt, dass die Aufnahmeeinheit an einem endständigen Roboterflansch der Gelenkarmrobotereinheit angeordnet ist. Der Gelenkarmroboter kann insbesondere automatisiert, teilautomatisiert oder manuell verfahren werden. Die Gelenkarmrobotereinheit kann vorzugsweise über eine Schnittstelle zur Befestigung der Aufnahmeeinheit als Endeffektor verfügen.

Der Begriff Auswerteeinheit meint vorliegend insbesondere eine Recheneinheit bzw. einen Prozessor, der eingerichtet ist, eine Abweichung zwischen einer Kontur in dem aufgenommenen Bild und einer Sollkontur zu bestimmen und auf Basis der Abweichung einen Verfahrbefehl für die Gelenkrobotereinheit zu bestimmen, um die Abweichung zu reduzieren, insbesondere zu minimieren.

Der Begriff Sollkontur meint vorliegend insbesondere einen Kreis. Bei einer Ausrichtung der Aufnahmeeinheit direkt oberhalb der zylindrischen Hülse, wobei die Aufnahmeeinheit und die zylindrische Hülse zueinander konzentrisch ausgerichtet bzw. koaxial/mit einer gemeinsamen Längsachse angeordnet sind, erscheint eine Kontur der zylindrischen Hülse auf dem Bild wie ein idealer Kreis. Mit anderen Worten ausgedrückt, führt jede Abweichung dieser konzentrischen Ausrichtung von Aufnahmeeinheit und zylindrischer Hülse zu einer Abweichung der Kontur von der Sollkontur. Die Kontur wird dann verzerrt dargestellt.

Der Begriff Verfahrbefehl meint vorliegend insbesondere einen oder mehrere Befehle zur Ansteuerung einer oder mehrerer Bewegungsachsen der Gelenkarmrobotereinheit.

Der Offenbarung liegt die Erkenntnis zugrunde, dass eine Aufnahmeeinheit, die ein Bild durch eine zylindrische Hülse aufnehmen soll, hierzu im Idealfall konzentrisch zu dieser ausgerichtet sein sollte. Das heißt, dass die Achse der zylindrischen Hülse und die Achse der Aufnahmeeinheit, in/mit der das Bild aufgenommen wird, somit in einer Flucht liegen. Bei einer Abweichung von diesem Idealfall, kommt zu einer Verzerrung des Bildes. Weiterhin wird ggfs. nur ein Teil des anatomischen Zielgebiets aufgenommen, da beide Achsen in einem zu großen Winkel zueinander bzw. zu schief zueinanderstehen und das Bild eher eine Innenfläche der zylindrischen Hülse abbildet als eine Bodenfläche der Hülse und das darunterliegende anatomische Zielgebiet.

Das offenbarungsgemäße Mikroskopiesystem löst dieses Problem, indem es vorteilhafterweise das aufgenommen Bild analysiert und eine Abweichung der aufgenommenen Kontur der zylindrischen Hülse von einer Sollkontur bestimmt. Dies kann beispielsweise über ein Bild-Analyse-Verfahren (z.B. Segmentierung, Merkmalserkennung) erfolgen. Auf Basis der bestimmten Abweichung kann eine Position der Aufnahmeeinheit ermittelt werden. Auf Basis dieser bestimmten Position kann rückgerechnet werden, wie die Aufnahmeeinheit bewegt werden muss, um die Abweichung zu minimieren. Hierzu werden dann Verfahrbefehle für die Gelenkarmrobotereinheit ermittelt, sodass die Aufnahmeeinheit in eine bessere Position, die eine Reduzierung der Abweichung ermöglicht, verfahren wird. Dies ermöglicht insgesamt ein besseres Bild, das keine Verzerrung darstellt. Weiterhin kann dadurch ein Chirurg besser in seiner Arbeit unterstützt werden. Weiterhin bedarf es hierfür keines Trackings der zylindrischen Hülse, um auf die Orientierung und Position der Hülse zu schließen. Dies führt insgesamt zu einer effizienten und einfachen Möglichkeit, eine genaue Ausrichtung des Mikroskopiesystems zu ermöglichen.

In einer bevorzugten Ausführungsform kann die Auswerteeinheit die Abweichung mittels eines Bild-Analyse-Verfahrens (bzw. Machine-Vision-Verfahrens bzw. mittels maschinellem Sehen) bestimmen.

Der Begriff Bild-Analyse-Verfahrens meint insbesondere automatisierte Verfahren, die geeignet sind, visuelle Informationen aus Bildern zu erfassen und zu interpretieren. Das Bild-Analyse-Verfahren kann eines oder mehrere der folgenden umfassen: Bildsegmentierung, Merkmalsextraktion, Objekterkennung und -klassifizierung, Mustererkennung. Auf diese Weise kann vorteilhafter die Abweichung sehr genau und ohne zusätzliche Hilfsmittel oder Zutun eines Menschen automatisiert bestimmt werden. Auf diese Weise kann vorteilhaft auf ein zusätzliches Tracking der Hülse verzichtet werden.

Vorzugsweise bestimmt die Auswerteeinheit die Abweichung mittels einer Bildsegmentierung.

Gemäß einer bevorzugten Ausführungsform kann die Sollkontur der Stirnseite der zylindrischen Hülse einem idealen Kreis entsprechen.

Der ideale Kreis kann einfacherweise durch einen konstanten Radius um einen Mittelpunkt beschrieben werden. Die Angabe des speziellen Radius ist zunächst nicht notwendig, da sich die Größe des Kreises mit dem Abstand zur Aufnahmeeinheit ändert, selbst wenn dieser bereits ideal ist. Somit kann über diese einfache Information, nämlich ein konstanter Radius, eine Sollkontur in dem Bild für die zylindrische Hülse definiert werden. Die Abweichung kann nun beispielsweise durch Bestimmung des Mittelpunkts und einer Auswertung des Radius erfolgen. Auf diese Weise können beispielsweise der kleinste Radius und der größte Radius ermittelt werden. Über eine derartige Auswertung kann beispielsweise eine Achse, um die die Gelenkarmrobotereinheit die Aufnahmeeinheit gedreht werden muss, um die Abweichung zu minimieren, ermittelt werden. Beispielsweise kann jeweils ein konstantes Inkrement gedreht werden, sodass bereits mit dieser einfachen Analyse eine Minimierung erreicht werden kann. Die Minimierung kann dabei iterativ solange erfolgen, bis die bestimmte Abweichung unter einen vorgegebenen Grenzwert liegt (z.B. 0,1% Abweichung vom idealen Kreis). Dies kann eine effiziente Ermittlung eines Verfahrbefehls zur Minimierung der Abweichung ermöglichen.

Gemäß einer bevorzugten Ausführungsform kann die Auswerteeinheit in dem aufgenommenen zumindest einen Teilabschnitt der Kontur der Stirnseite eine erste Achse und eine zur ersten Achse senkrecht verlaufende zweite Achse bestimmen, wobei die erste Achse und die zweite Achse durch einen Mittelpunkt der Kontur der Stirnseite verlaufen,wobei die erste Achse so gewählt ist, dass sie eine maximale Länge innerhalb der Kontur der Stirnseite aufweist, wobei der Verfahrbefehl eine Drehung um die erste Achse umfasst. Das heißt, dass eine Drehachse durch die erste Achse gebildet ist.

Der Mittelpunkt, die erste Achse und die zweite Achse können jeweils wiederum mit einem Bild-Analyse-Verfahren bzw. Machine-Vision-Verfahren ermittelt werden. Die auf diese Weise bestimmte erste Achse gibt Aufschluss über eine virtuelle Achse, um die der Gelenkarmroboter die Aufnahmeeinheit herumdrehen muss, um die Abweichung zu minimieren. Auf Basis dieser virtuellen Achse können Verfahrbefehle für den Gelenkarmroboter abgeleitet werden, die die Aufnahmeeinheit um diese virtuelle Achse herumdrehen. Anders ausgedrückt, entsprechen die erste Achse und die zweite Achse den beiden Hauptachsen der Ellipse.

Hierzu kann über die Aufnahmeeinheit ein Abstand zwischen der Aufnahmeeinheit und dem Mittelpunkt des anatomischen Zielgebiets ermittelt werden. Die Aufnahmeeinheit kann ein Stereomikroskop sein, das diese Information (i.e. Tiefeninformationen) bereitstellt. Weiterhin kann hier die Begebenheit genutzt werden, dass eine virtuelle Linie zwischen dem Mittelpunkt der Aufnahmeeinheit (i.e. Endeffektor) und dem Mittelpunkt der Kontur mit der zweiten Achse der Kontur zusammenfällt. Weiterhin kann beispielsweise nun über die erste Achse bei konstantem Abstand um ein vorbestimmtes Winkelinkrement in einer Kreisbahn um den Mittelpunkt in Richtung der zweiten Achse herumgedreht werden. Über die beiden Positionen (Mittelpunkt Kontur, Position Endeffektor), Drehachse (i.e. erste Achse). Drehrichtung (i.e. zweite Achse) und vorbestimmtes Winkelinkrement kann eine Position des Endeffektors im Raum bestimmt werden. Diese Position wird dann in Verfahrbefehle (i.e. Drehbewegungen der einzelnen Gelenkarme) übersetzt und den Verfahrachsen zur Verfügung gestellt. Das Winkelinkrement kann dabei beispielsweise fest sein, 0,1°, 1°, 2° etc. Alternativ kann aus dem Verhältnis der Längen der ersten Achse und der zweiten Achse der Kontur sowie der Positionen vom Mittelpunkt der Kontur und des Endeffektors ein Winkel bestimmt werden, um den gedreht werden muss, sodass die Aufnahmeeinheit und die zylindrische Hülse zueinander ausgerichtet sind.

Gemäß einer bevorzugten Ausführungsform kann die Auswerteeinheit eingerichtet sein, in dem Bild eine Innenfläche der Kontur des Zugangsports zu bestimmen und auf Basis der bestimmten Innenfläche eine Richtung für die Drehung um die erste Achse für den Verfahrbefehl zu bestimmen. Das heißt, dass eine Drehrichtung durch die erste Achse gebildet ist.

Beispielsweise kann die Auswerteeinheit über ein Bild-Analyse-Verfahren bzw. Machine-Vision-Verfahren, beispielsweise eine Segmentierung, eine Innenfläche einer zylindrischen Hülse identifizieren. Mit dieser Information kann beispielsweise eine Drehrichtung für die Drehung der Aufnahmeeinheit um die erste Achse ermittelt werden. Beispielsweise kann aus der Innenfläche, insbesondere einer Größeninformation der Innenfläche, die sich um den Umfang der Kontur verteilt, die Drehrichtung zur Reduzierung bzw. Minimierung der Abweichung abgeleitet werden. Beispielsweise entspricht die Drehrichtung der Richtung in eine größere Innenfläche identifiziert werden kann.

Gemäß einer bevorzugten Ausführungsform kann die Auswerteeinheit eingerichtet sein, eine Abweichung des Mittelpunkts des aufgenommenen zumindest einen Teilabschnitts der Kontur der Stirnseite von einem Mittelpunkt des Bildes zu bestimmen; wobei der Verfahrbefehl eine Bewegung umfasst, sodass der Mittelpunkt des aufgenommenen zumindest einen Teilabschnitts der Kontur der Stirnseite und der Mittelpunkt des Bildes übereinstimmen. Mit anderen Worten kann die Auswerteeinheit eingerichtet sein, eine translatorische Abweichung der Kontur der Stirnseite bzw. der Hülse im Bild zu bestimmen/berechnen, wobei der Verfahrbefehl eine translatorische Bewegung in Richtung einer Bildmitte umfasst bzw. derart, dass das Bild in der Hülse in der Bildmitte ist.

Gemäß einer bevorzugten Ausführungsform kann das Mikroskopiesystem eine Navigationseinheit (bzw. ein Navigationssystem bzw. eine
Positionsbestimmungseinheit) zur Erfassung einer Position der Aufnahmeeinheit und/oder einer Position eines Patienten umfassen.

Die Navigationseinheit kann dabei beispielsweise eine Kameraeinheit zur Bestimmung einer Pose bzw. Position und/oder Orientierung der Aufnahmeeinheit und/oder einer Pose bzw. Position und/oder Orientierung des Patienten. Die Position kann dabei beispielsweise vorteilhafterweise ohne Marker an der Aufnahmeeinheit und/oder dem Patienten über ein Bild-Analyse-Verfahren bzw. Machine-Vision-Verfahren erfolgen. Die Navigationseinheit kann alternativ beispielsweise einen Marker, der am Patienten angeordnet ist, zur Positionsbestimmung tracken. Die Navigationseinheit kann alternativ beispielsweise einen Marker, der an der Aufnahmeeinheit angeordnet ist, zur Positionsbestimmung tracken. Mit Hilfe der Position der Aufnahmeeinheit und/oder der Position des Patienten kann ein Modell des anatomisches Zielgebiets (z.B. CT-Bild, MRT-Bild), das beispielsweise neben dem zeitaktuellen Bild angezeigt wird, entsprechend ausgerichtet werden. Dies kann sich positiv auf einen chirurgischen Eingriff auswirken, wenn sowohl die zeitaktuelle Aufnahme des anatomisches Zielgebiets als auch ein korrespondierendes Modell des anatomisches Zielgebiets nebeneinander sowie vorzugsweise zueinander ausgerichtet angezeigt werden.

Gemäß einer bevorzugten Ausführungsform kann das Mikroskopiesystem eine Anzeigeeinheit umfassen, die eingerichtet ist, das Bild anzuzeigen.

Durch die Anzeigeeinheit kann beispielsweise vorteilhafterweise das Bild angezeigt werden. Dadurch kann sich vorteilhafterweise eine Effizienz des chirurgischen Eingriffs erhöhen. Die Anzeigeeinheit kann einen Bildschirm umfassen.

Gemäß einer bevorzugten Ausführungsform kann die Anzeigeeinheit eingerichtet ist, ein Modell des anatomisches Zielgebiets und der zylindrischen Hülse anzuzeigen, insbesondere wobei das Bild und das Modell zueinander ausgerichtet sind.

Das Modell kann ein präoperatives Bild umfassen, beispielsweise ein MRT-Bild oder ein CT-Bild.

Auf diese Weise kann der Chirurg bei seiner Tätigkeit effizient unterstützt werden, da sowohl ein qualitativ hochwertiges zeitaktuelles Bild als auch ein Modell hierzu in ausgerichteter Position angezeigt werden können.

Gemäß einer bevorzugten Ausführungsform kann das medizinische Mikroskopiesystem eine Eingabeeinheit umfassen, über die die Ausrichtung der Aufnahmeeinheit startbar ist und/oder kontinuierlich erfolgt.

Die Eingabeeinheit kann vorliegend ein Schalter, ein HMI (Human-Machine-Interface) oder Touchdisplay sein. Durch die Eingabeeinheit kann vorteilhaft der Ausrichtprozess ausgelöst bzw. getriggert werden, sodass dieser erst durchgeführt wird, wenn dieser wirklich notwendig ist. Dies kann vorteilhafterweise ressourcenschonend sein.

Gemäß einer bevorzugten Ausführungsform kann die Auswerteeinheit die Bestimmung der Abweichung auf Basis von Geometriedaten der zylindrischen Hülse durchführen.

Die Geometriedaten können einen Radius, eine Höhe, eine Wandstärke der zylindrischen Hülse umfassen. Vorzugsweise umfassen die Geometriedaten einen Radius. Die Geometriedaten können beispielsweise in einem internen Speicher des Mikroskopiesystems abgelegt sein oder in einem externen Speicher, auf den das Mirkroskopiesystem zurückgreift. Durch die Geometriedaten kann die Genauigkeit der Ausrichtung weiter verbessert werden.

Gemäß einer bevorzugten Ausführungsform kann die Aufnahmeeinheit eingerichtet sein, während des Ausrichtens automatisch einen Zoomvorgang durchzuführen, sodass die Abweichung besser bestimmt werden kann.

Durch den Zoomvorgang kann die Kontur im Bild vergrößert oder verkleinert werden, sodass eine Kontur die Kontur vollständig sichtbar und/oder analysierbar sind. Der Zoomvorgang kann mithilfe Geometriedaten der zylindrischen Hülse weiter optimiert werden. Weiterhin kann beispielsweise eine vorbestimmte Regel verwendet werden, um den Zoomvorgang zu automatisieren. Beispielsweise kann vorgegeben werden, dass eine im Bild ermittelte Kontur einen bestimmten Anteil, beispielsweise 95%, des Bildes ausmachen soll. Dadurch kann der manuelle Einstellaufwand für einen Nutzer bei gleichzeitiger Erhöhung der Qualität des Ausrichtens und des korrespondierenden Bildes erhöht werden.

Gemäß einer bevorzugten Ausführungsform kann die Aufnahmeeinheit eingerichtet sein, nach dem Ausrichten automatisch einen Zoomvorgang durchzuführen, sodass das anatomische Zielgebiet vergrößert aufgenommen wird.

Durch die Vergrößerung des anatomisches Zielgebiets bei gleichzeitig ausgerichteter Aufnahme kann dem Chirurgen vorteilhafterweise ein qualitativ hochwertiges Bild des anatomisches Zielgebiets zeitaktuell angezeigt werden.

Gemäß einer bevorzugten Ausführungsform kann die zylindrische Hülse ein (optisches) Muster aufweisen. Das Muster dient insbesondere zum Nachverfolgen der Hülse durch eine (optische) Kamera mittels maschinellem Sehen
Das Muster kann beispielsweise an der Stirnseite der zylindrischen Hülse, an einer äußeren Mantelfläche der zylindrischen und/oder einer inneren Mantelfläche der zylindrischen Hülse angebracht sein. Das Muster meint vorliegend ein dem Bild-Analyse-Verfahren bekanntes Muster. Auf diese Weise kann die Effizienz und Genauigkeit der Bestimmung der Abweichung des zumindest einen Teilabschnitts der Kontur der Stirnseite von einer Sollkontur der Stirnseite der zylindrischen Hülse erhöht werden.

Gemäß einer bevorzugten Ausführungsform kann die Auswerteeinheit eingerichtet sein, geometrische Daten der zylindrischen Hülse zu empfangen und auf Basis der geometrischen Daten der zylindrischen Hülse in dem aufgenommenen Bild eine Abweichung des zumindest einen Teilabschnitts der Kontur der Stirnseite von einer Sollkontur einer Stirnseite der zylindrischen Hülse zu bestimmen.

Die geometrischen Daten meinen dabei beispielsweise die den Außenradius, den Innenradius und/oder die Länge der zylindrischen Hülse. Auf diese Weise kann die Effizienz und Genauigkeit der Bestimmung der Abweichung des zumindest einen Teilabschnitts der Kontur der Stirnseite von einer Sollkontur der Stirnseite der zylindrischen Hülse erhöht werden.

Ein weiterer Aspekt der vorliegenden Offenbarung bezieht sich auf eine Verwendung eines einer Gelenkarmrobotereinheit und/oder einer Aufnahmeeinheit in einem oben näher beschriebenen medizinischen Mikroskopiesystem.

Die Einheiten gemäß einem oder mehreren Ausführungsbeispielen können unter Verwendung von Hardware, Software und/oder einer Kombination davon implementiert werden. Die Einheiten können einteilig oder mehrteilig sein. Hardware-Einheiten können beispielsweise durch Verarbeitungsschaltungen wie einen Prozessor, eine Zentraleinheit (CPU), einen Controller, eine arithmetische Logikeinheit (ALU), einen digitalen Signalprozessor, einen Mikrocomputer, ein feldprogrammierbares Gate-Array (FPGA), ein System-on-Chip (SoC), eine programmierbare Logikeinheit, einen Mikroprozessor oder jede andere Vorrichtung implementiert werden, die in der Lage ist, auf Befehle zu reagieren und diese in einer festgelegten Weise auszuführen.

Die Einheiten können eine oder mehrere Schnittstellenschaltungen umfassen. In einigen Beispielen können die Schnittstellenschaltungen verdrahtete oder drahtlose Schnittstellen umfassen, die mit einem lokalen Netz (LAN), dem Internet, einem Weitverkehrsnetz (WAN) oder Kombinationen davon verbunden sind. Die Funktionalität einer bestimmten Einheit der vorliegenden Offenbarung kann auf mehrere Einheiten verteilt werden, die über Schnittstellenschaltungen verbunden sind.

Die Einheiten gemäß einem oder mehreren Ausführungsbeispielen können auch ein oder mehrere Speichergeräte umfassen. Bei der einen oder den mehreren Speichervorrichtungen kann es sich um materielle oder nichttransitorische computerlesbare Speichermedien handeln, wie Direktzugriffsspeicher (RAM), Festwertspeicher (ROM), ein permanentes Massenspeichergerät (z. B. ein Festplattenlaufwerk), ein Solid-State-Gerät (z. B. NAND Flash) und/oder jeder andere Datenspeichermechanismus, der Daten speichern und aufzeichnen kann. Die eine oder mehrere Speichervorrichtungen können zum Speichern von Computerprogramme, Programmcode, Anweisungen oder eine Kombination davon zu speichern.

Die hier beschriebenen Erläuterungen und Vorteile einzelner Ausführungsformen gelten sinngemäß auch für die anderen Ausführungsformen. Verschiedene beispielhafte Merkmale der Ausführungsformen können erfindungsgemäß kombiniert werden, wo immer dies technisch sinnvoll und machbar ist.

In diesem Zusammenhang hat sich nun herausgestellt, dass ein weiterer Bedarf besteht, ein medizinisches Mikroskopiesystem zur Aufnahme eines anatomischen Zielgebiets eines Patienten in einem medizinischen Operationsbereich bereitzustellen.

### Kurzbeschreibung der Figuren

Fig. 1 ist eine Darstellung eines Mikroskopiesystems zur Aufnahme eines anatomischen Zielgebiets eines Patienten in einem medizinischen Operationsbereich der vorliegenden Offenbarung;
Fig. 2 zeigt ein Bild mit einer eingezeichneten Kontur einer zylindrischen Hülse;
Fig. 3 zeigt ein weiteres Bild einer eingezeichneten Kontur einer zylindrischen Hülse;
Fig. 4 zeigt eine schematische Analyse einer Kontur einer zylindrischen Hülse;
Fig. 5 zeigt ein weiteres Bild mit einer Innenfläche einer Kontur einer zylindrischen Hülse;
Fig. 6 zeigt eine ausgerichtete Aufnahmeeinheit im Vergleich zu einer nicht ausgerichteten Aufnahmeeinheit.

### Beschreibung der Ausführungsbeispiele

Nachstehend werden Ausführungsbeispiele der vorliegenden Offenbarung auf der Basis der zugehörigen Figuren beschrieben.

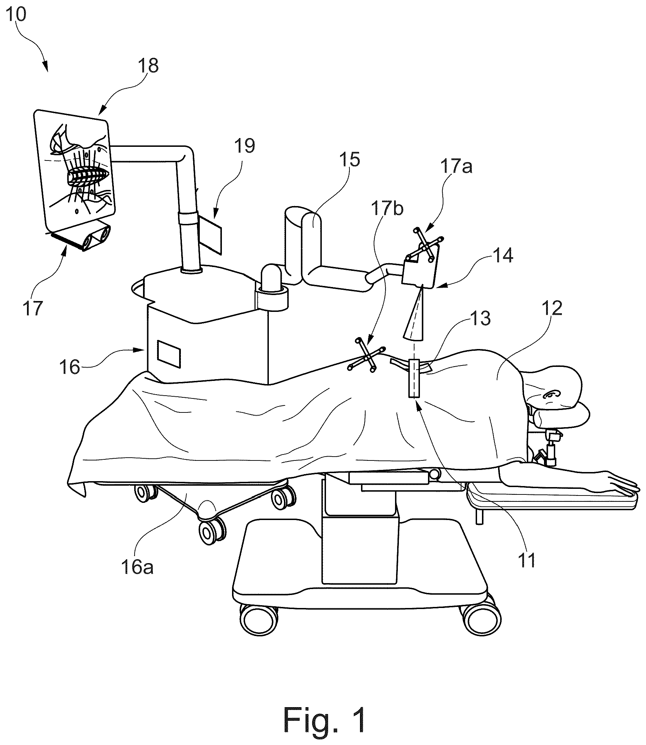

Fig. 1 zeigt ein Mikroskopiesystem 10 zur Aufnahme eines anatomischen Zielgebiets 11 eines Patienten 12 in einem medizinischen Operationsbereich.

Das Mikroskopiesystem 10 weist eine zylindrische Hülse 13 für einen Zugangsport auf. Oberhalb des anatomisches Zielgebiets 11 ist die zylindrische Hülse 13 angeordnet. Insbesondere kann die zylindrische Hülse 13 eingerichtet sein, um auf oder außerhalb/oberhalb einer Objektoberfläche angeordnet zu werden. Durch die zylindrische Hülse 13 kann ein Chirurg beispielsweise mit einem medizinischen Werkzeug wie einem Skalpell auf das anatomische Zielgebiet, z. B. ein Tumor, einwirken.

Das medizinische Mikroskopiesystem 10 umfasst eine Aufnahmeeinheit 14. Die Aufnahmeeinheit 14 ist eingerichtet, ein Bild des anatomisches Zielgebiets 11 durch die zylindrische Hülse 13 aufzunehmen. Die Aufnahmeeinheit 14 ist vorliegend ein Stereomikroskop. Das Bild umfasst dabei zumindest einen Teilabschnitt einer Kontur einer Stirnseite der zylindrischen Hülse 13.

Das medizinische Mikroskopiesystem 10 weist eine Gelenkarmrobotereinheit 15 auf. Die Aufnahmeeinheit 14 ist vorliegend an der Gelenkarmrobotereinheit 15 angeordnet. Die Gelenkarmrobotereinheit 15 ist vorliegend an einem Trägerwagen 16a angeordnet.

Das medizinische Mikroskopiesystem 10 umfasst weiter eine Auswerteeinheit 16. Die Auswerteeinheit 16 ist vorliegend eine Steuerung, die im Trägerwagen 16a angeordnet ist. Die Auswerteeinheit 16 ist eingerichtet, in dem aufgenommenen Bild eine Abweichung des zumindest einen Teilabschnitts der Kontur der Stirnseite von einer Sollkontur einer Stirnseite der zylindrischen Hülse 13 zu bestimmen. Die Sollkontur entspricht dabei vorzugsweise bei einer ideal ausgerichteten Anordnung der Aufnahmeeinheit 14 oberhalb der zylindrischen Hülse 13 einem (idealen) Kreis.

Die Auswerteeinheit 16 bestimmt die Abweichung vorzugsweise mit Hilfe eines Bild-Analyse-Verfahrens bzw. Machine-Vision-Verfahrens. Hierzu ermittelt sie eine erste Achse durch einen Mittelpunkt der Kontur und eine zweite senkrecht zur ersten Achse verlaufende Achse. Die erste Achse wird dabei so bestimmt, dass sie eine maximale Länge aufweist. Bei einem idealen Kreis, wären beiden Achsen gleich lang. Sofern jedoch die Aufnahmeeinheit 14 nicht konzentrisch zu einer Längsachse der zylindrischen Hülse 13 angeordnet ist, sind die beiden Achsen nicht gleich lang. Es liegt somit eine Verzerrung vor. Diese Verzerrung hat zur Folge, dass der untere Bereich der zylindrischen Hülse 13 und somit das anatomische Zielgebiet 11 nicht bestmöglich auf dem Bild erfasst werden.

Die Auswerteeinheit 16 ist weiterhin eingerichtet, auf Basis der bestimmten Abweichung einen Verfahrbefehl zur Korrektur einer Position der Gelenkarmrobotereinheit zu bestimmen, sodass die Abweichung reduziert wird. Die Auswerteeinheit 16 bestimmt vorliegend über die Positionen (Mittelpunkt Kontur, Position Endeffektor), Drehachse (i.e. erste Achse), Drehrichtung (i.e. zweite Achse) und ein vorbestimmtes Winkelinkrement eine Position des Endeffektors im Raum. Die Auswerteeinheit 16 leitet dann aus dieser Position Verfahrbefehle (i.e. Drehbewegungen der einzelnen Gelenkarme) für die Gelenkarmrobotereinheit 15 ab und stellt diese der Gelenkarmrobotereinheit 15 zur Verfügung.

Die Gelenkarmrobotereinheit 15 verfährt dann entsprechend dem Verfahrbefehl eine oder mehrere Bewegungsachsen, um die Aufnahmeeinheit 14 entsprechend auszurichten, sodass die Abweichung minimiert wird. Dieser Vorgang kann iterativ durchlaufen werden, bis keine Abweichung mehr vorliegt oder die Abweichung kleiner als ein Grenzwert ist.

Alternativ kann die Auswerteeinheit 16 eingerichtet sein, aus dem Verhältnis der Längen der ersten Achse und der zweiten Achse der Kontur sowie der Positionen vom Mittelpunkt der Kontur und des Endeffektors ein Winkel zu bestimmten, um den die Aufnahmeeinheit gedreht werden muss, sodass die Aufnahmeeinheit und die zylindrische Hülse 13 zueinander ausgerichtet sind.

Das Mikroskopiesystem 10 umfasst vorliegend weiter eine Navigationseinheit 17 zur Erfassung einer Position der Aufnahmeeinheit 14 und/oder einer Position eines Patienten 12. Hierzu sind Markierungen 17a und 17b an Aufnahmeeinheit 14 und Patient 12 angebracht. Mit Hilfe der Positionen kann beispielsweise ein Modell des anatomisches Zielgebiets 11 zu dem aufgenommenen Bild des anatomisches Zielgebiets an einer Anzeigeeinheit 18 ausgerichtet werden. Die Anzeigeeinheit 18 kann dabei beispielsweise das aufgenommene Bild sowie das Modell nebeneinander anzeigen.

Weiterhin umfasst das Mikroskopiesystem 10 eine Eingabeeinheit 19, über die der Ausrichtung der Aufnahmeeinheit 14 manuell gestartet werden kann.

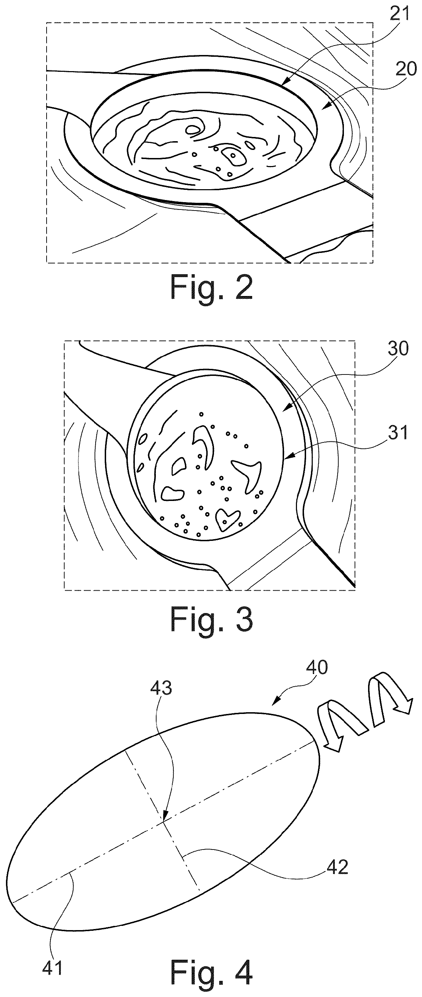

Fig. 2 zeigt ein aufgenommenes Bild durch eine oben näher beschriebene Aufnahmeeinheit. In dem Bild sind vorliegend die zylindrische Hülse 20 sowie eine eingezeichnete Kontur 21 der Stirnseite der zylindrischen Hülse 20 zu sehen. Es ist deutlich zu erkennen, dass die Kontur 21 verzerrt dargestellt ist, da die Aufnahmeeinheit 14 und die zylindrische Hülse 20 nicht zueinander ausgerichtet sind.

Fig. 3 zeigt ein aufgenommenes Bild durch eine oben näher beschriebene Aufnahmeeinheit. Im Bild sind vorliegend die zylindrische Hülse 30 sowie eine eingezeichnete Kontur 31 der Stirnseite der zylindrische Hülse 30 zu sehen. Es ist deutlich zu erkennen, dass die Kontur 31 nicht verzerrt dargestellt ist, da die Aufnahmeeinheit 14 und die zylindrische Hülse 30 zueinander ausgerichtet sind.

Fig. 4 zeigt eine schematische Analyse einer Kontur 40 einer zylindrischen Hülse. Darin sind jeweils die erste Achse 41 und senkrecht zu dieser die zweite Achse 42 eingezeichnet. Beide Achsen verlaufen durch den Mittelpunkt 43. Die Aufnahmeeinheit muss vorliegend um die erste Achse 41, i.e. die längste Achse, in Richtung der zweiten Achse 42 gedreht werden, um die Verzerrung zu reduzieren.

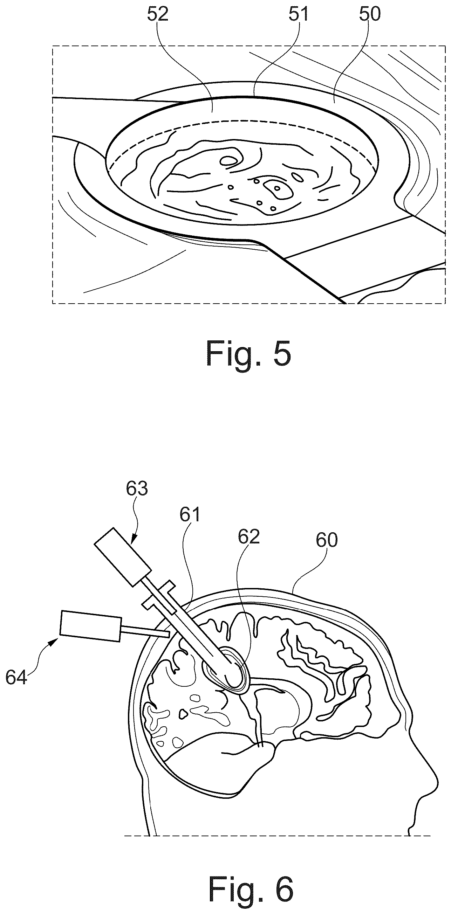

Fig. 5 zeigt ein Bild mit einer eingezeichneten Innenkontur 52 sowie Kontur 51 der Stirnseite einer zylindrischen Hülse 50. Die Innenkontur 52 gibt hier Aufschluss über die die Drehrichtung, vorliegend in die Bildebene hinein, in welche die Aufnahmeeinheit gedreht werden muss, um eine Abweichung zu reduzieren.

Fig. 6. zeigt eine ausgerichtete Aufnahmeeinheit 63 im Vergleich zu einer nicht ausgerichteten Aufnahmeeinheit 64. Dabei ist vorliegend das anatomische Zielgebiet 62 ein Tumor im Kopf eines Patienten 60. Die zylindrische Hülse 61 ragt dabei teilweise in den Kopf des Patienten 60 hinein. Die Aufnahmeeinheit 64 ist vorliegend schräg zu einer Längsachse der zylindrischen Hülse 61 angeordnet. Mithilfe des oben näher beschriebenen Mikroskopiesystems konnte die nicht ausgerichtete Aufnahmeeinheit 64 in eine ausgerichtete Position gebracht werden.

### Bezugszeichenliste

- 10: Medizinisches Mikroskopiesystem
- 11, 62: Anatomische Zielgebiet
- 12, 60: Patienten
- 13, 20, 30, 50, 61: Zylindrische Hülse
- 14, 63, 64: Aufnahmeeinheit
- 15: Gelenkarmrobotereinheit
- 16: Auswerteeinheit
- 21, 31, 40, 51: Kontur
- 41: Erste Achse
- 42: Zweite Achse
- 43: Mittelpunkt
- 52: Innenfläche der Kontur
- 17: Navigationseinheit
- 17a: Markierung Aufnahmeeinheit
- 17b: Markierung Patient
- 18: Anzeigeeinheit
- 19: Eingabeeinheit

## Patentansprüche

1. Medizinisches Mikroskopiesystem (10) zur Aufnahme eines anatomischen Zielgebiets (11, 62) eines Patienten (12) in einem medizinischen Operationsbereich, wobei oberhalb des anatomischen Zielgebiets (11, 62) eine zylindrische Hülse (13, 20, 30, 50, 61) angeordnet ist, mit
einer Aufnahmeeinheit (14, 63, 64), die eingerichtet ist, ein Bild des anatomisches Zielgebiets (11, 62) durch die zylindrische Hülse (13, 20, 30, 50, 61) aufzunehmen,
wobei das Bild zumindest einen Teilabschnitt einer Kontur (21, 31, 40, 51) einer Stirnseite der zylindrischen Hülse (13, 20, 30, 50, 61) umfasst,
einer Gelenkarmrobotereinheit (15), wobei die Aufnahmeeinheit (14, 63, 64) an der Gelenkarmrobotereinheit (15) angeordnet ist,
einer Auswerteeinheit (16),
wobei die Auswerteeinheit (16) eingerichtet ist, in dem aufgenommenen Bild eine Abweichung des zumindest einen Teilabschnitts der Kontur (21, 31, 40, 51) der Stirnseite von einer Sollkontur einer Stirnseite der zylindrischen Hülse (13, 20, 30, 50, 61) zu bestimmen,
wobei die Auswerteeinheit (16) eingerichtet ist, auf Basis der bestimmten Abweichung einen Verfahrbefehl zur Korrektur einer Pose der Gelenkarmrobotereinheit (15) zu bestimmen, sodass die Abweichung reduziert wird, und
wobei die Gelenkarmrobotereinheit (15) eingerichtet ist, zumindest eine Achse (41, 42) der Gelenkarmrobotereinheit (15) auf Basis des bestimmten Verfahrbefehls zu bewegen, um eine Ausrichtung der Aufnahmeeinheit (14, 63, 64) zu ermöglichen, sodass die Abweichung reduziert wird.

2. Medizinisches Mikroskopiesystem (10) nach Anspruch 1, wobei die Auswerteeinheit (16) die Abweichung mittels eines Bild-Analyse-Verfahrens bestimmt.

3. Medizinisches Mikroskopiesystem (10) nach Anspruch 1 oder 2, wobei die Sollkontur der Stirnseite der zylindrischen Hülse (13, 20, 30, 50, 61) einem idealen Kreis entspricht.

4. Medizinisches Mikroskopiesystem (10) nach einem der vorherigen Ansprüche,
wobei die Auswerteeinheit (16) in dem aufgenommenen zumindest einen Teilabschnitt der Kontur (21, 31, 40, 51) der Stirnseite eine erste Achse (41) und eine zur ersten Achse senkrecht verlaufende zweite Achse (42) bestimmt;
wobei die erste Achse (41) und die zweite Achse (42) durch einen Mittelpunkt (43) der Kontur (21, 31, 40, 51) der Stirnseite verlaufen;
wobei die erste Achse (41) so gewählt ist, dass sie eine maximale Länge innerhalb der Kontur (21, 31, 40, 51) der Stirnseite aufweist; und
wobei der Verfahrbefehl eine Drehung um die erste Achse (41) umfasst.

5. Medizinisches Mikroskopiesystem (10) nach Anspruch 4,
wobei die Auswerteeinheit (16) eingerichtet ist, in dem Bild eine Innenfläche der Kontur (52) der zylindrischen Hülse (13, 20, 30, 50, 61) zu bestimmen, und auf Basis der bestimmten Innenfläche (52) eine Richtung für die Drehung um die erste Achse (41) für den Verfahrbefehl zu bestimmen.

6. Medizinisches Mikroskopiesystem (10) nach einem der vorherigen Ansprüche, wobei die Auswerteeinheit (16) eingerichtet ist, eine Abweichung des Mittelpunkts des aufgenommenen zumindest einen Teilabschnitts der Kontur (21, 31, 40, 51) der Stirnseite von einem Mittelpunkt des Bildes zu bestimmen;
wobei der Verfahrbefehl eine Bewegung umfasst, sodass der Mittelpunkt des aufgenommenen zumindest einen Teilabschnitts der Kontur (21, 31, 40, 51) der Stirnseite und der Mittelpunkt des Bildes übereinstimmen.

7. Medizinisches Mikroskopiesystem (10) nach einem der vorherigen Ansprüche, weiter umfassend eine Navigationseinheit (17) zur Erfassung einer Position der Aufnahmeeinheit (14, 63, 64) und/oder einer Position eines Patienten (12).

8. Medizinisches Mikroskopiesystem (10) nach einem der vorherigen Ansprüche, weiter umfassend eine Anzeigeeinheit (18), die eingerichtet ist, das Bild anzuzeigen.

9. Medizinisches Mikroskopiesystem (10) nach einem der vorherigen Ansprüche, wobei die Anzeigeeinheit (18) eingerichtet ist, ein Modell des anatomisches Zielgebiets (11, 62) und der zylindrischen Hülse (13, 20, 30, 50, 61) anzuzeigen, insbesondere wobei das Bild und das Modell zueinander ausgerichtet sind.

10. Medizinisches Mikroskopiesystem (10) nach einem der vorherigen Ansprüche, weiter umfassend eine Eingabeeinheit (19), über die die Ausrichtung der Aufnahmeeinheit (14, 63, 64) startbar ist und/oder kontinuierlich erfolgt.

11. Medizinisches Mikroskopiesystem (10) nach einem der vorherigen Ansprüche, wobei die Auswerteeinheit (16) die Bestimmung der Abweichung auf Basis von Geometriedaten der zylindrischen Hülse (13, 20, 30, 50, 61) durchführt.

12. Medizinisches Mikroskopiesystem (10) nach einem der vorherigen Ansprüche, wobei die Aufnahmeeinheit (14, 63, 64) eingerichtet ist, während des Ausrichtens automatisch einen Zoomvorgang durchzuführen, sodass die Abweichung besser bestimmt werden kann.

13. Medizinisches Mikroskopiesystem (10) nach einem der vorherigen Ansprüche, wobei die Aufnahmeeinheit (14, 63, 64) eingerichtet ist, nach dem Ausrichten automatisch einen Zoomvorgang durchzuführen, sodass das anatomische Zielgebiet vergrößert aufgenommen wird.

14. Medizinisches Mikroskopiesystem (10) nach einem der vorherigen Ansprüche, wobei die zylindrische Hülse (13, 20, 30, 50, 61) ein Muster aufweist.

15. Medizinisches Mikroskopiesystem (10) nach einem der vorherigen Ansprüche, wobei die Auswerteeinheit (16) eingerichtet ist, geometrische Daten der zylindrischen Hülse (13, 20, 30, 50, 61) zu empfangen und auf Basis der geometrischen Daten der zylindrischen Hülse in dem aufgenommenen Bild eine Abweichung des zumindest einen Teilabschnitts der Kontur (21, 31, 40, 51) der Stirnseite von einer Sollkontur einer Stirnseite der zylindrischen Hülse (13, 20, 30, 50, 61) zu bestimmen

16. Verwendung eines einer Gelenkarmeinheit und/oder einer Aufnahmeeinheit (14, 63, 64) in einem Medizinisches Mikroskopiesystem (10) nach einem der Ansprüche 1 bis 15.
